# EUROPEAN PATENT SPECIFICATION

(11) **EP 1 283 043 B1**
(45) Date of publication and mention of the grant of the patent: **09.01.2008**
(21) Application number: 01932088.6
(22) Date of filing: 16.05.2001
(51) Int. Cl.: A61K 9/08, A61K 31/436, A61K 31/4164, A61K 31/137, A61K 45/06, A61P 9/08, A61P 27/14

(54) **OPHTHALMIC SOLUTION**
OPHTHALMISCHE LÖSUNG
COMPOSITION OPHTALMIQUE

(30) Priority: 17.05.2000 JP 2000145641
(43) Date of publication of application: 12.02.2003
(73) Proprietor: Senju Pharmaceutical Co., Ltd., Osaka-shi, Osaka 541-0046 (JP)
(72) Inventor: NAKAYAMA, Hisayuki, Nishinomiya-shi, Hyogo 662-0826 (JP); NISHIHATA, Shuichi, Toyonaka-shi, Osaka 560-0082 (JP); AKI, Hiroshi, Kobe-shi, Hyogo 657-0034 (JP)
(74) Representative: von Kreisler, Alek
(86) International application number: PCT/JP2001/004084
(87) International publication number: WO 2001/087304

(56) References cited:
- WO-A-95/07103
- JP-A- 3 128 332
- JP-A- 4 327 533
- US-A- 4 607 038
- OGAWA TAKAHIRO ET AL.: 'Effects of pre-instilled mydreatics on the intraocular concentration and anti-inflammatory action of topical 0.1% pranoprofen. (2). Study on multiple dosing' NIPPON GANKA GAKKAI ZASSHI vol. 96, no. 9, 1992, pages 1079 - 1084, XP002944175
- OGAWA TAKAHIRO ET AL.: 'Effects of pre-instilled mydreatics on the intraocular concentration and anti-inflammatory action of topical 0.1% pranoprofen' NIPPON GANKA GAKKAI ZASSHI vol. 92, no. 9, 1988, pages 1510 - 1519, XP002944176

## Description

### Technical Field

The present invention relates to an ophthalmic solution that removes or relieves congestion in outer ocular areas, particularly in conjunctiva and pericorneal area.

### Background Art

Bulbar conjunctiva (white of eye) easily congests by physical or chemical irritation, such as light ray, scald, cigarette smoke, vapor of chemical substance and the like. In addition, blepharitis, conjunctivitis and the like also cause congestion. To remove these congestions, commercially available ophthalmic solutions containing a vasoconstrictor are used and they afford temporary effects. However, pericorneal injection accompanying severe congestion in the pericorneal area is caused by inflammations in cornea, sclera and uvea, and instillation of vasoconstrictor cannot remove the congestion. Even instillation of an ophthalmic solution containing an antiphlogistic, such as allantoin, dipotassium glycyrrhizinate and the like, and a vasoconstrictor, such as naphazoline hydrochloride and the like, is insufficient to remove or relieve the congestion caused by these inflammations.

### Disclosure of the Invention

A major object of the present invention is to provide an ophthalmic solution effective for removing or relieving the congestion in outer ocular areas (particularly conjunctiva and pericorneal area).

As a result of intensive studies conducted by the present inventors in an attempt to achieve the above-mentioned object, it has been found that an ophthalmic solution containing pranoprofen (chemical name: α-methyl-5H-[1]benzopyrano[2,3-b]pyridine-7-acetic acid) or a pharmacologically acceptable salt thereof, and a vasoconstrictor removes or dramatically relieves congestion in the outer ocular area, which resulted in the completion of the present invention.

Accordingly, the present invention relates to the following.
(1) An ophthalmic solution containing pranoprofen or a pharmacologically acceptable salt thereof, and a vasoconstrictor.
(2) The ophthalmic solution according to the above-mentioned (1), wherein the pranoprofen or a pharmacologically acceptable salt thereof is contained at a concentration of pranoprofen of 0.01-2.0 w/v%, and the vasoconstrictor at a concentration of 0.0005-0.1 w/v%.
(3) The ophthalmic solution according to the above-mentioned (1) or (2), wherein the vasoconstrictor is at least one kind selected from the group consisting of naphazoline, tetrahydrozoline, phenylephrine and pharmacologically acceptable salts thereof.
(4) The ophthalmic solution according to the above-mentioned (1), which is an agent for ameliorating congestion in an outer ocular area.
(5) A fortifier of an ameliorating action of a vasoconstrictor on congestion in an outer ocular area, which contains pranoprofen or a pharmacologically acceptable salt thereof as an active ingredient.
(6) The fortifier according to the above-mentioned (5), wherein the vasoconstrictor is at least one kind selected from the group consisting of naphazoline, tetrahydrozoline, phenylephrine and pharmacologically acceptable salts thereof.
(7) A method for ameliorating congestion in an outer ocular area, which comprises administering an effective amount of pranoprofen or a pharmacologically acceptable salt thereof and an effective amount of a vasoconstrictor to a patient.
(8) The method according to the above-mentioned (7), wherein the vasoconstrictor is at least one kind selected from the group consisting of naphazoline, tetrahydrozoline, phenylephrine and pharmacologically acceptable salts thereof.
(9) Use of pranoprofen or a pharmacologically acceptable salt thereof, and a vasoconstrictor for the production of an agent for ameliorating congestion in an outer ocular area.
(10) The use according to the above-mentioned (9), wherein the agent contains pranoprofen or a pharmacologically acceptable salt thereof at a concentration of pranoprofen of 0.01-2.0 w/v% and the vasoconstrictor at a concentration of 0.0005-0.1 w/v%.
(11) The use according to the above-mentioned (9) or (10), wherein the vasoconstrictor is at least one kind selected from the group consisting of naphazoline, tetrahydrozoline, phenylephrine and pharmacologically acceptable salts thereof.
(12) Use of pranoprofen or a pharmacologically acceptable salt thereof for the production of a fortifier of an ameliorating action of a vasoconstrictor on congestion in an outer ocular area.
(13) The use according to the above-mentioned (12), wherein the vasoconstrictor is at least one kind selected from the group consisting of naphazoline, tetrahydrozoline, phenylephrine and pharmacologically acceptable salts thereof.
(14) A commercial package comprising the ophthalmic solution according to the above-mentioned (4) and a written matter associated therewith, the written matter stating that the ophthalmic solution can or should be used for ameliorating congestion in an outer ocular area.
(15) A commercial package comprising the fortifier of an ameliorating action of a vasoconstrictor on congestion in an outer ocular area according to the above-mentioned (5) and a written matter associated therewith, the written matter stating that the fortifier can or should be used for enhancing the ameliorating action of the vasoconstrictor on congestion in an outer ocular area.

### Detailed Description of the Invention

The present invention provides an ophthalmic solution containing pranoprofen or a pharmacologically acceptable salt thereof, and a vasoconstrictor. The present invention also provides a fortifier of an ameliorating action of a vasoconstrictor on congestion in an outer ocular area, which contains pranoprofen or a pharmacologically acceptable salt thereof as an active ingredient, and which can potentiate an ameliorating action possessed by a vasoconstrictor to be mentioned below on congestion in an outer ocular area, when it is used concurrently with the vasoconstrictor. In the following, the ophthalmic solution and the fortifier of the present invention are also collectively referred to as a "preparation of the present invention".

In the present invention, the potentiation of an ameliorating action of a vasoconstrictor on congestion in an outer ocular area means a significantly improved removal or relief action on congestion in an outer ocular area that the vasoconstrictor has, which is achieved by concurrent use of the fortifier of the present invention, as compared to the use of the vasoconstrictor alone.

The vasoconstrictor to be used in the present invention is exemplified by naphazoline, tetrahydrozoline, phenylephrine, epinephrine, ephedrine, methylephedrine, pharmacologically acceptable salts thereof and the like, which may be used alone or in combination of two or more kinds thereof. Of these, naphazoline, tetrahydrozoline and phenylephrine are more preferable. Examples of the pharmacologically acceptable salt include hydrochlorides and nitrates. While the concentration of a vasoconstrictor may be determined appropriately depending on various properties of the pharmaceutical agents, it is generally 0.0005-0.1 w/v%.

The dosage form of the ophthalmic solution of the present invention may be, for example, an aqueous solution, a suspension, an emulsion and the like, with preference given to an aqueous solution.

The fortifier of the present invention may take a dosage form of an eye ointment or an ophthalmic solution. The dosage form of the ophthalmic solution is exemplified by those mentioned above, with preference given to an aqueous solution.

In the present invention, a pharmacologically acceptable salt of pranoprofen is exemplified by, but not limited to, metal salts such as sodium salt, potassium salt, calcium salt, magnesium salt, aluminum salt and the like, and salts with an organic base such as triethylamine, diethylamine, morpholine, piperazine and the like.

The content of pranoprofen or a pharmacologically acceptable salt thereof in the preparation of the present invention as pranoprofen is generally 0.01-2.0 w/v%, preferably 0.05-1.0 w/v%, as a standard content, from which it may be increased or decreased appropriately according to the object of use.

The preparation of the present invention may contain, besides the above-mentioned ingredients, optional ingredients as necessary. For example, antiphlogistics such as dipotassium glycyrrhizinate, allantoin, ε-aminocaproic acid and the like, vitamins such as flavin adenin dinucleotide sodium, cyanocobalamin, pyridoxine hydrochloride, tocopherol acetate and the like, amino acids such as aspartic acid, aminoethylsulfonic acid and the like, and anticholinesterase agents such as neostigmine methylsulfate and the like may be added as necessary.

The preparation of the present invention may further contain additives such as buffer, isotonicity agent, dissolution aids, preservative, viscous base, chelating agent and refrigerant, as appropriate.

Examples of the buffer include phosphate buffer, borate buffer, citrate buffer, tartrate buffer, acetate buffer, amino acid and the like.

Examples of the isotonicity agent include saccharides such as sorbitol, glucose, mannitol and the like, polyhydric alcohols such as glycerin, propylene glycol and the like, salts such as sodium chloride and the like, boric acid and the like.

Examples of the dissolution aids include nonionic surfactants such as polyoxyethylene sorbitan monooleate, polyoxyethylene hydrogenated castor oil, tyloxapol, pluronic and the like, polyhydric alcohols such as glycerin, macrogol and the like, and the like.

Examples of the preservative include quaternary ammonium salts such as benzalkonium chloride, benzethonium chloride, cetylpyridinium chloride and the like, parahydroxybenzoic acid esters such as methyl parahydroxybenzoate, ethyl parahydroxybenzoate, propyl parahydroxybenzoate, butyl parahydroxybenzoate and the like, benzyl alcohol, sorbic acid, thimerosal, chlorobutanol, sodium dehydroacetate and the like.

Examples of the viscous base include water-soluble polymers such as polyvinylpyrrolidone, polyethylene glycol, polyvinyl alcohol and the like, celluloses such as hydroxyethylcellulose, methylcellulose, hydroxypropylmethylcellulose, carboxymethylcellulose sodium and the like, and the like.

Examples of the chelating agent include sodium edetate, citric acid and the like and examples of the refrigerant include *l*-menthol, borneol, camphor, eucalyptus oil and the like.

The pH of the preparation of the present invention is generally adjusted to 6.0-8.5, preferably about 7.0-8.0.

The ophthalmic solution of the present invention may be produced according to a preparation method known *per se,* for example, a method described in The Japanese Pharmacopoeia thirteenth Edition, General Rules for Preparations. For example, a buffer, an isotonicity agent, a preservative and other additives are dissolved in water, then a vasoconstrictor and pranoprofen are dissolved and sterilized to produce the ophthalmic solution of the present invention.

The fortifier of the present invention can be produced according to a preparation method known *per se* depending on the dosage form thereof. For example, it can be produced according to a method described in The Japanese Pharmacopoeia thirteenth Edition, General Rules for Preparations.

The present invention further provides a method for ameliorating congestion in an outer ocular area, which comprises administering an effective amount of pranoprofen or a pharmacologically acceptable salt thereof, and an effective amount of a vasoconstrictor to a patient. In the method of the present invention, pranoprofen or a pharmacologically acceptable salt thereof and a vasoconstrictor may be prepared as separate preparations and administered, or may be prepared into a single preparation containing the both ingredients and administered.

In the present invention, the "outer ocular area" refers to, from among the components constituting adnexa oculi, areas facing the body surface, as generally used in the ophthalmologic field, and specifically includes eyelid, conjunctiva, cornea, anterior sclera and the like. In the present invention, moreover, the ameliorating of the congestion in an outer ocular area means removal or relief of congestion in an outer ocular area, particularly congestion in the conjunctiva and pericorneal area.

For the method for ameliorating congestion in an outer ocular area according to the present invention, the preparation of the present invention produced as mentioned above can be used. When the ophthalmic solution of the present invention is used, the dose thereof may be any as long as it is sufficient to afford an effect of relieving the congestion. In the case of an ophthalmic solution containing naphazoline hydrochloride in a proportion of 0.003 w/v% and pranoprofen in a proportion of 0.05 w/v%, 0.05-0.1 ml thereof is given 3-5 times a day by instillation into the eye, whereby congestion in the outer ocular area can be removed or remarkably relieved.

### Examples

The present invention is described in more detail in the following by means of Experimental Examples and Examples, which are not to be construed as limitative.

### Experimental Example 1: Effect on congestion in an outer ocular area of rabbit

The ophthalmic solutions A-E containing pranoprofen and naphazoline hydrochloride or tetrahydrozoline hydrochloride as a vasoconstrictor and having the formulation shown in Table 1 were prepared by a conventional method. A 0.1% sodium arachidonate solution (50 µl) was instilled into the eye of a rabbit once to induce congestion and edema of ocular mucosa, followed by observation. At one hour prior to induction, each ophthalmic solution (50 µl) was instilled once into the eye of the rabbit, and 5 min, 10 min and 60 min after induction, the degree of congestion was evaluated in 5 levels shown in Table 2 and the degree of edema was evaluated in 6 levels shown in Table 3 (n=5). The results of the degrees of congestion and edema (average of 5 rabbits) are shown in Tables 4 and 5, respectively.

**Table 1**

| | A | B | C | D | E |
|---|---|---|---|---|---|
| pranoprofen | 0.05 g | - | - | 0.05 g | 0.05 g |
| naphazoline hydrochloride | - | 0.003g | - | 0.003g | - |
| tetrahydrozoline hydrochloride | - | - | 0.05 g | - | 0.05 g |
| boric acid borax | 1.6 g q.s. | 1.6 g q.s. | 1.6 g q.s. | 1.6 g q.s. | 1.6 g q.s. |
| sterile purified water | q.s. | q.s. | q.s. | q.s. | q.s. |
| total amount pH | 100 ml 7.5 | 100 ml 7.5 | 100 ml 7.5 | 100 ml 7.5 | 100 ml 7.5 |

**Table 2**

| Level of congestion | Score |
|---|---|
| No congestion | 0 |
| Slight expansion of pericornel blood vessel | 1 |
| Clear expansion of pericornel blood vessel | 2 |
| Marked expansion of pericornel blood vessel | 3 |
| | |
| Further expansion of pericornel blood vessel causing beefy red | 4 |

**Table 3**

| Degree of edema | Score |
|---|---|
| No swelling | 0 |
| Slight level of edema observed | 1 |
| Somewhat swollen than normal | 2 |
| Marked swelling | 3 |
| Swollen and drooped on eyelid | 4 |
| Swollen to cause eversion | 5 |

**Table 4**

| Formulation | 5 min after instillation | 10 min after instillation | 60 min after instillation |
|---|---|---|---|
| A | 3.6 | 3.6 | 3.0 |
| B | 3.2 | 3.2 | 3.4 |
| C | 2.4 | 2.4 | 2.0 |
| D | 0.8 | 0.2 | 0.6 |
| E | 1.2 | 1.2 | 0.8 |

**Table 5**

| Formulation | 5 min after instillation | 10 min after instillation | 60 min after instillation |
|---|---|---|---|
| A | 3.0 | 3.0 | 3.0 |
| B | 2.8 | 3.0 | 3.0 |
| C | 3.0 | 3.0 | 2.4 |
| D | 0.2 | 0.2 | 0 |
| E | 0.2 | 0.2 | 0 |

As is clear from the results shown in Table 4, pranoprofen, naphazoline hydrochloride and tetrahydrozoline hydrochloride used alone did not show a congestion relief effect. In contrast, ophthalmic solutions D and E containing pranoprofen and a vasoconstrictor showed a superior effect of ameliorating the congestion in the outer ocular area from immediately after induction.

As regards the degree of edema shown in Table 5, pranoprofen, naphazoline hydrochloride and tetrahydrozoline hydrochloride used alone showed marked swelling even 60 min after induction, but ophthalmic solutions D and E containing pranoprofen and a vasoconstrictor did not show edema from immediately after induction.

### Example 1

| | |
|---|---|
| Pranoprofen | 0.05 g |
| tetrahydrozoline hydrochloride | 0.05 g |
| boric acid | 1.6 g |
| borax | q.s. |
| benzalkonium chloride | 0.005 g |
| polysorbate 80 | 0.1 g |
| sterile purified water | ad 100 ml |

Polysorbate 80, boric acid, tetrahydrozoline hydrochloride, pranoprofen and benzalkonium chloride are successively dissolved in sterile purified water (ca. 70 ml) and adjusted to pH 7.0 with borax, after which sterile purified water is added to the total amount of 100 ml. This solution is sterilized by filtration and filled in a vessel for ophthalmic solution.

### Example 2

| | |
|---|---|
| pranoprofen | 0.05 g |
| phenylephrine hydrochloride | 0.1 g |
| sodium dihydrogen phosphate | 0.2 g |
| sodium chloride | 0.8 g |
| benzalkonium chloride | 0.005 g |
| polysorbate 80 | 0.1 g |
| sodium hydroxide | q.s. |
| sterile purified water | ad 100 ml |

Polysorbate 80, sodium dihydrogen phosphate, sodium chloride, phenylephrine hydrochloride, pranoprofen and benzalkonium chloride are successively dissolved in sterile purified water (ca. 70 ml) and adjusted to pH 7.5 with sodium hydroxide, after which sterile purified water is added to the total amount of 100 ml. This solution is sterilized by filtration and filled in a vessel for ophthalmic solution.

### Example 3

| | |
|---|---|
| pranoprofen | 0.05 g |
| naphazoline hydrochloride | 0.003 g |
| sodium chloride | 0.8 g |
| citric acid ' | 0.2 g |
| borax | q.s. |
| benzalkonium chloride | 0.005 g |
| polysorbate 80 | 0.1 g |
| sterile purified water | ad 100 ml |

Polysorbate 80, citric acid, sodium chloride, naphazoline hydrochloride, pranoprofen and benzalkonium chloride are successively dissolved in sterile purified water (ca. 70 ml) and adjusted to pH 7.8 with borax, after which sterile purified water is added to the total amount of 100 ml. This solution is sterilized by filtration and filled in a vessel for ophthalmic solution.

### Example 4

| | |
|---|---|
| pranoprofen | 0.05 g |
| naphazoline hydrochloride | 0.003 g |
| neostigmine methylsulfate | 0.005 g |
| sodium chloride | 0.8 g |
| boric acid | 0.2 g |
| benzethonium chloride | 0.005 g |
| polysorbate 80 | 0.1 g |
| sodium hydroxide | q.s. |
| sterile purified water | ad 100 ml |

Polysorbate 80, boric acid, sodium chloride, neostigmine methylsulfate, naphazoline hydrochloride, pranoprofen and benzethonium chloride are successively dissolved in sterile purified water (ca. 70 ml) and adjusted to pH 7.5 with sodium hydroxide, after which sterile purified water is added to the total amount of 100 ml. This solution is sterilized by filtration and filled in a vessel for ophthalmic solution.

### Example 5

| | |
|---|---|
| sodium salt of pranoprofen | 0.055 g |
| naphazoline hydrochloride | 0.003 g |
| sodium chloride | 0.8 g |
| boric acid | 0.2 g |
| benzethonium chloride | 0.005 g |
| polysorbate 80 | 0.1 g |
| sodium hydroxide | q.s. |
| sterile purified water | ad 100 ml |

Polysorbate 80, boric acid, sodium chloride, naphazoline hydrochloride, sodium salt of pranoprofen and benzethonium chloride are successively dissolved in sterile purified water (ca. 70 ml) and adjusted to pH 7.5 with sodium hydroxide, after which sterile purified water is added to the total amount of 100 ml. This solution is sterilized by filtration and filled in a vessel for ophthalmic solution.

### Example 6

| | |
|---|---|
| pranoprofen | 0.05 g |
| naphazoline hydrochloride | 0.003 g |
| sodium chloride | 0.8 g |
| citric acid | 0.2 g |
| borax | q.s. |
| sorbic acid | 0.1 g |
| 1-menthol | 0.002 g |
| polysorbate 80 | 0.1 g |
| sterile purified water | ad 100 ml |

Citric acid, sodium chloride, sorbic acid, naphazoline hydrochloride and pranoprofen are successively dissolved in sterile purified water (ca. 70 ml) and adjusted to pH 7.3 with borax. To the obtained solution is added *l*-menthol dispersed in polysorbate 80, and sterile purified water is added to the total amount of 100 ml. This solution is sterilized by filtration and filled in a vessel for ophthalmic solution.

### Industrial Applicability

By the combined use of pranoprofen or a pharmacologically acceptable salt thereof, and a vasoconstrictor, the congestion in an outer ocular area can be effectively removed or relieved.

This application is based on a patent application No. 2000-145641 filed in Japan, the contents of which are hereby incorporated by reference.

## Claims

1. An ophthalmic solution for ameliorating congestion in an outer ocular area, comprising pranoprofen or a pharmacologically acceptable salt thereof at a concentration of pranoprofen of 0.01-2.0 w/v%, and a vasoconstrictor at a concentration of 0.0005-0.1 w/v%.

2. The ophthalmic solution according to claim 1, wherein the vasoconstrictor is at least one kind selected from the group consisting of naphazoline, tetrahydrozoline, phenylephrine and pharmacologically acceptable salts thereof.

3. Use of pranoprofen or a pharmacologically acceptable salt thereof, and a vasoconstrictor for the production of an agent for ameliorating congestion in an outer ocular area.

4. The use according to claim 3, wherein the agent contains pranoprofen or a pharmacologically acceptable salt thereof at a concentration of pranoprofen of 0.01-2.0 w/v% and the vasoconstrictor at a concentration of 0.0005-0.1 w/v%.

5. The use according to claim3 or 4, wherein the vasoconstrictor is at least one kind selected from the group consisting of naphazoline, tetrahydrozoline, phenylephrine and pharmacologically acceptable salts thereof.

## Patentansprüche

1. Ophthalmische Lösung zur Verbesserung der Kongestion in einem äußeren Augenbereich, umfassend Pranoprofen oder ein pharmakologisch annehmbares Salz davon in einer Pranoprofen-Konzentration von 0,01 bis 2,0% (w/v) und ein gefäßverengendes Mittel in einer Konzentration von 0,0005 bis 0,1% (w/v).

2. Ophthalmische Lösung gemäß Anspruch 1, wobei es sich bei dem gefäßverengenden Mittel um wenigstens eine Art handelt, die aus der Gruppe ausgewählt ist, die aus Naphazolin, Tetrahydrozolin, Phenylephrin und pharmakologisch annehmbaren Salzen davon besteht.

3. Verwendung von Pranoprofen oder eines pharmakologisch annehmbaren Salzes davon und eines gefäßverengenden Mittels zur Herstellung eines Mittels zur Verbesserung der Kongestion in einem äußeren Augenbereich.

4. Verwendung gemäß Anspruch 3, wobei das Mittel Pranoprofen oder ein pharmakologisch annehmbares Salz davon in einer Pranoprofen-Konzentration von 0,01 bis 2,0% (w/v) und das gefäßverengende Mittel in einer Konzentration von 0,0005 bis 0,1% (w/v) enthält.

5. Verwendung gemäß Anspruch 3 oder 4, wobei es sich bei dem gefäßverengenden Mittel um wenigstens eine Art handelt, die aus der Gruppe ausgewählt ist, die aus Naphazolin, Tetrahydrozolin, Phenylephrin und pharmakologisch annehmbaren Salzen davon besteht.

## Revendications

1. Solution ophtalmique pour l'amélioration de la congestion dans une zone oculaire extérieure, comprenant du pranoprofène ou un sel de celui-ci pharmacologiquement acceptable à une concentration en pranoprofène de 0,01 à 2,0 % en poids/vol. et un vasoconstricteur à une concentration de 0,0005 à 0,1 % en poids/vol.

2. Solution ophtalmique suivant la revendication 1, dans laquelle le vasoconstricteur est au moins une espèce sélectionnée dans le groupe comprenant la naphazoline, la tétrahydrozoline, la phényléphrine et leurs sels pharmacologiquement acceptables.

3. Utilisation de pranoprofène ou d'un seul pharmacologiquement acceptable de celui-ci et d'un vasoconstricteur pour la production d'un agent pour l'amélioration de la congestion dans une zone oculaire extérieure.

4. Utilisation suivant la revendication 3, dans laquelle l'agent contient du pranoprofène ou un sel pharmacologiquement acceptable de celui-ci à une concentration en pranoprofène de 0,01 à 2,0 % en poids/vol. et le vasoconstricteur à une concentration de 0,0005 à 0,1 % en poids/vol.

5. Utilisation suivant la revendication 3 ou 4, dans laquelle le vasoconstricteur est au moins une espèce sélectionnée dans le groupe comprenant la naphazoline, la tétrahydrozoline, la phényléphrine et leurs sels pharmacologiquement acceptables.
